Europäisches Patentamt

European Patent Office

Office européen des brevets

(11) Publication number: **0 408 783 A1**

# EUROPEAN PATENT APPLICATION

(21) Application number: 89113200.3

(51) Int. Cl.⁵: **A61N 1/36**

(22) Date of filing: **19.07.89**

(43) Date of publication of application:
**23.01.91 Bulletin 91/04**

(84) Designated Contracting States:
**BE CH DE FR GB IT LI SE**

(71) Applicant: **FUTURE MEDICAL PRODUCTS, INC.**

**Hauppague New York(US)**

(72) Inventor: **Eckerson, Harold D.**

**Sag Harbor N.Y.(US)**

(74) Representative: **Pfenning, Meinig & Partnerner**
**Kurfürstendamm 170**
**D-1000 Berlin 15(DE)**

(54) **Method and apparatus for drug free neurostimulation.**

(57) A method and device adapted to give people suffering from drug or alcohol withdrawal relief from severe physical symptoms associated with initial abstinence from the drug to which they are addicted. The method and device delivers a non-invasive and very specific electromagnetic waveform (106) to the patient's mastoid regions behind the ear, and may be worn on a belt. The device has electrode means (302, 304) for applying a train of electrical pulses (106) to the mastoid regions (306) of a patient, and means (200, 202, 204, 206, 208, 210) for generating a train of electrical pulses (106) to be input to said electrode means (302, 304).

Figure 2

The present invention relates generally to the field of drug and alcohol withdrawal symptom alleviation, and, more specifically, to the method and use of biologically applied electrical signals to relieve suffering from narcotic withdrawal.

Drug and alcohol addiction is a major problem throughout the world. Some sources quote 10% of the U.S. population as being addicted to either drugs or alcohol. The problem is becoming worse and is fast becoming a political as well as an economic and medical issue. In addition, addictions frequently result from medically-indicated opiate and analgesic administration to individuals who suffer from chronic pain. The biochemical basis of addiction involves the body's natural opiate-like pain killers which are secreted by the brain cells into the nervous system. These have become known generically as the endorphins and the enkephalins. Many different types have been identified to date and they interact in complex ways, which have yet to be fully elucidated, with neurons. In structure and function these endorphins resemble the opiate drugs of addiction, like heroin and cocaine, and are also known to be involved in all compulsive addictive type behaviours, including alcoholism, smoking, and overeating.

Heroin addicts, for example, take into their bodies an exogenous source of opiates which severely interferes with the endorphin/enkephalin system. When this external source is interrupted, severe withdrawal symptoms ensue. This withdrawal state has been associated with low levels of the body's normal endogenous endorphins and altered ratios of different types of endorphins and enkephalins. This has been caused by the previous involvement of the heroin or other drug of addiction in the physiological processes. It is this low and abnormally functioning endorphin level that causes the symptoms of acute withdrawal. This withdrawal state persists until the body has had a chance to normalize this system which takes on average 3-4 days. This acute withdrawal state if left untreated is known as going "cold turkey".

Standard methods of getting patients through this withdrawal state, i.e., detoxifying them, generally use the principle of drug substitution and gradual weaning from the substitute drug, e.g. methadone in the case of heroin, and Valium in the case of alcoholics. Many addicts only achieve the substitution phase and never get weaned off methadone, hence the large number of methadone clinics in the United States and Europe. The best that these patients can expect is that they may be able to function better and live longer on methadone than on heroin. The vast majority of addicts return to their addictions following detoxification, hence the persistent and relentless nature of the problem.

Withdrawal symptoms consist of two main types corresponding with the two major subdivisions of the human nervous system. Symptoms are associated with the somatosensory nervous system and the autonomic nervous system. The somatosensory nervous system is the main one of which we are aware which senses our environment, position and well being of our bodies, and also senses pain. The autonomic nervous system controls the "automatic" functions of life, i.e. the functions of organs like the heart, sweat glands, etc. Withdrawal symptoms associated with the somatonervous system include joint pains and muscle cramps which can be extremely severe. The autonomic symptoms include nausea, vomiting, cold sweats, gooseflesh, and signs such as markedly dilated pupils. A third group of symptoms common to withdrawal from all addictions and compulsive behaviour includes anxiety and craving.

It is, therefore, a feature of certain embodiments of the present invention to provide a drug free neurostimulation method and device (NSD) that provides a 3 to 5 day treatment designed to precipitously reduce both acute and chronic withdrawal symptomatology of all addictive chemical substances, without drugs and with virtually no negative side effects. The NSD acts by specific electrical frequency stimulation of endorphin production that has previously been decreased due to chronic substance abuse.

Another feature of another embodiment is to provide a drug free neurostimulation method and device (NSD) that is compact and is affixed to the belt of a patient, and is utilized intermittently on a daily basis for approximately 3 to 5 days. The NSD unit is designed for simple application by physicians, nurses and patients.

A still further feature of another embodiment is to provide a drug free neurostimulation method and device (NSD) that can be completely programmed to either treat a particular drug or addiction of a combination of simultaneous addictions, and requires minimal supervision by a physician or nurse.

A further feature of another embodiment is to provide a drug free neurostimulation method and device (NSD) that can be used in conjunction with the administration of opiates and all other analgesics to individuals suffering from chronic pain, so that the amount of analgesic pharmaceuticals required by the patient may be reduced, thereby minimizing complications arising from chronic analgesic therapy, including mental apathy, confusion, narcotism and dependence.

A still further feature of another embodiment is to provide a drug free neurostimulation method and device (NSD) that can be used in order to treat individuals suffering from such symptoms as stress, insomnia and endogenous depression, so that the abnormal chemical neurotransmitters may be beneficially affected/regulated/normalized.

The following classification of the principal withdrawal symptoms that patients experience after prolonged use of narcotic agents or a mixture of narcotics and alcohol:

Type 1 . Central nervous system (somatosensory) symptoms such as restlessness, irritability, muscle aches, yawning and severe, prolonged insomnia;

Type 2 . Symptoms mediated through autonomic nervous system, such as running nose, over-breathing, abdominal cramps, vomiting and diarrhea; and,

Type 3 . Two symptoms common to all drugs of dependence: anxiety and craving for the drug.

The NSD Unit electrodes are placed behind the ear on the mastoid processes in close proximity to stimulate a branch of the vagus nerve, a major component of the autonomic nervous system.

A still further feature of another aspect of this invention, is to provide a drug free neurostimulation method and device (NSD) that by retrograde stimulation similar in principle to electro-acupuncture, alleviates Type 2 symptoms.

A yet further feature of another aspect is to provide a drug free neurostimulation method and device (NSD) that alleviates Type 1 symptoms by using electromagnetic waves to stimulate the brain cells to restart their normal production of endorphins which have, in the addict, been altered or repressed.

Another further feature of another aspect is to provide a drug free neurostimulation method and device (NSD) that alleviates Type 3 symptoms by a probable direct affect on the brain, in particular, on a part of the brain termed the Corpus Striatum which contains the majority of opiate receptors that, when interfered with electrochemically by the neurostimulator, alleviate symptoms of anxiety and craving.

It is a still further feature of certain embodiments to provide a drug free neurostimulation method and device (NSD) that produces a symptomless, shorter detoxification process and a lower recidivism rate.

A still further feature is to provide a drug free neurostimulation method and device (NSD) that delivers a very specific electromagnetic waveform known to be effective in this case for narcotic, or drug addiction, used with or without alcohol.

In accordance with one aspect of the present invention, there is provided a method of relieving a patient's suffering due to narcotic and alcohol withdrawal, which comprises the steps of (a) applying a train of electrical pulses to the patient's mastoid processes; and (b) controlling the waveform and frequency of said pulses.

In accordance with another aspect of this invention, there is provided a drug free neurostimulator, which comprises the steps of (a) an electrode harness for applying a train of electrical pulses to the mastoid processes of a patient; and (b) means for generating a train of electrical pulses to be input to said electrode harness, wherein by the application of said pulses to said mastoid processes, a patient's suffering due to narcotic and alcohol withdrawal is relieved.

Application of the present invention to patients who have just entered the withdrawal phase of detoxification following abstinence from their drug of addiction, causes a cessation of all these symptoms in a period of about 40 minutes.

The specific low frequency electromagnetic waveform affects the production and function of the endorphins and enkephalins in such a way as to return them to normal and alleviate the symptoms of acute withdrawal. Clinical data using the invention has shown that it can alleviate severe withdrawal symptoms in a period of about 40 minutes. The patient then uses the device for a period of about 3 to 5 days, basically deciding the amount of time the person requires the device to be active for. During this period patients do not require any other forms of medication to deal with their withdrawal symptoms. As a result, the patient experiences a painless detoxification process and is rendered drug free, and clinical data suggests that the recidivism rate in patients treated by this method may be much lower than the rate of recidivism in patients treated by the standard therapeutic approach.

In the device of the present invention, the highly specific waveform is generated from a battery-operated, calculator-sized generator which is attached to the patient's belt. The generator is attached by two leads to two surface electrodes which are placed on the patient's mastoid processes behind the ears. Following a period of about 40 minutes the symptoms associated with acute narcotic withdrawal are alleviated or significantly reduced.

The device of the present invention is worn by a patient during the initial 3 to 5 days of abstinence. Patients may alter the intensity of stimulation themselves, and in general, they decide the length of the periods of stimulation they require each day. During periods of stimulation the patient feels no discomfort and is totally mobile.

The electrodes of the device of the present invention do not pierce the patient's skin and so there is no chance of infection or transmission of blood-borne infectious diseases. In addition to reducing the physical symptoms of withdrawal, the present invention is known to decrease the total time of the clinical and sub-clinical withdrawal state associated with initial abstinence from drugs of dependence. This is observed by comparing the withdrawal period associated with use of the present invention to the withdrawal period associated with standard therapies, such as methadone substitution therapy.

The present invention may be embodied in the form illustrated in the accompanying drawings, attention being called to the fact, however, that the drawings are illustrative only and that changes may be made in the specific construction illustrated without departing from the spirit and scope of the invention.

In the drawings,

Figure 1 depicts the electrode harness in place on a patient's head in the process of neurostimulation;

Figure 2 depicts the electrode harness connected to the output of the circuitry;

Figure 2a is an exploded side view of one of the electrodes from the wire snap-on connector;

Figure 2b is an exploded front view of one of the electrodes from the retainer hole in the tension-band;

Figure 3 is an electronic block diagram of the circuitry involved in the present invention;

Figure 4 is an electronic schematic diagram showing crystal oscillator and divider, and trimmer components of a preferred embodiment;

Figure 5 is an electronic schematic diagram showing the cycling-pulse generator component of a preferred embodiment;

Figure 6 is an electronic schematic diagram showing the waveform network component of a preferred embodiment;

Figure 7a illustrates the voltage present at the output pin 3 of the cycling-pulse generator depicted in Figure 5;

Figure 7b illustrates the voltage present across capacitor C4 of the waveform network depicted in Figure 6;

Figure 8 illustrates the voltage present at output pin 1 of the circuit depicted in Figure 4;

Figure 9 illustrates the voltage present after capacitor C6 of the circuit depicted in Figure 6;

Figure 10 illustrates the output waveform; and

Figure 11 is a block diagram showing a typical assortment of monitoring functions to assist in standardized application of the circuitry in patient care.

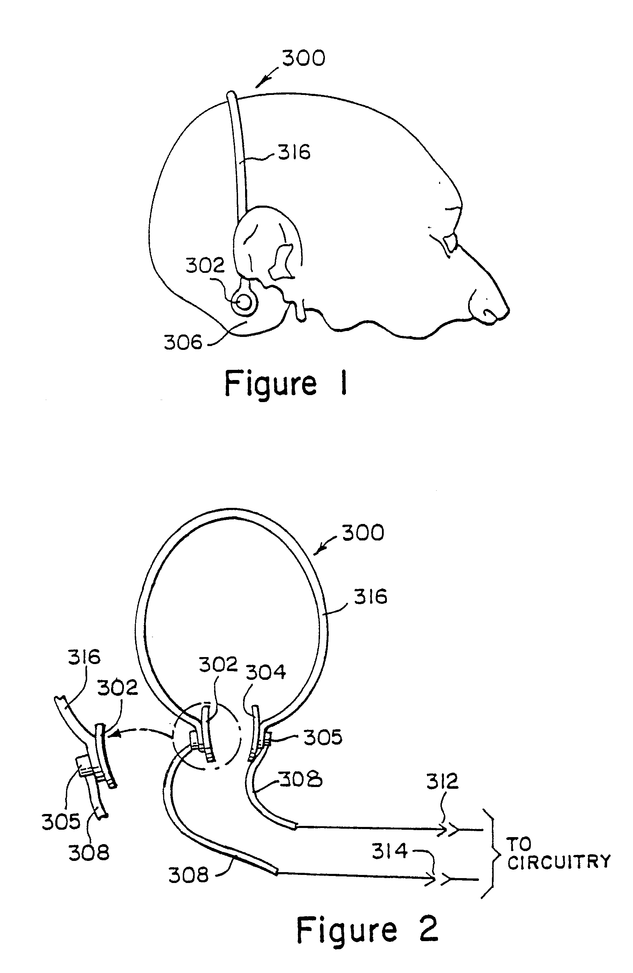

Referring to Figures 1 to 2b, the electrode harness 300 places electrodes 302 and 304 against the patient's mastoid processes 306, typified to wire snap-on connector 305 on 306. Electrodes 302 and 304 are connected by wires 308 and 310 to the output of the circuitry via plugs 312 and 314, respectively. Electrodes 302 and 304 are held in place by tension-band 316.

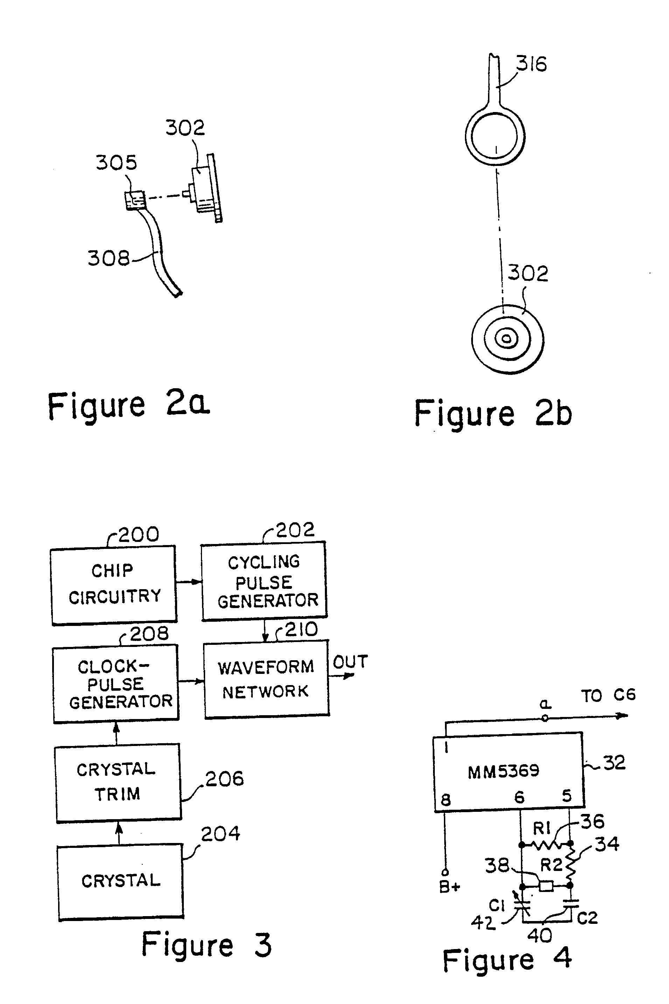

The generation of the therapeutic electrical waveform is best understood with reference to Figures 3 to 10. The apparatus shown is a preferred embodiment for generating the requisite waveform, however, other circuit configurations are possible within the scope of the present invention.

The electronic block diagram of Figure 3 combines all functional blocks illustrated in detail in Figures 4, 5, and 6.

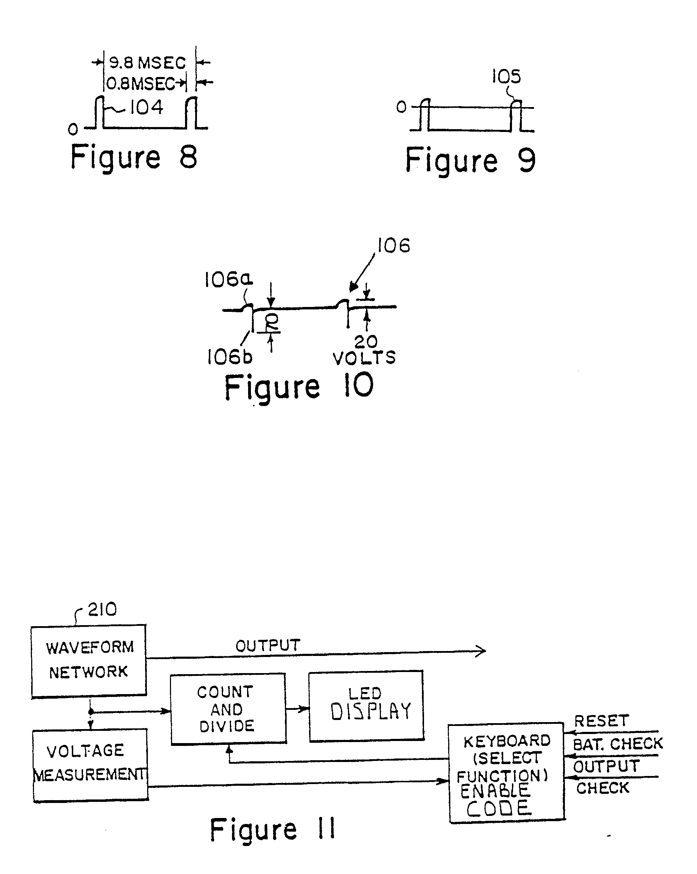

The circuit illustrated in Figure 4 is used to generate the voltage illustrated in Figure 8 (104), e.g., $T = 9.8$, $f = 102$ Hz. An oscillator/divider chip 32, typified by the commercially available chip MM5369, uses a piezoelectric crystal 38 as well as resistors R1 (36) and R2 (34) and capacitors C2 (40) and C1 (42). The repetition rate of the positive pulse train from the output at pin 1 may be varied by adjusting the trim circuitry (C1 is shown as a variable capacitor) or by changing the frequency of crystal 38. Typically, the pulse width at the output is 1 millisecond, and the repetition rate is 100 Hertz. The crystal shown as numeral 38 in Figure 4 is represented as crystal 204 in Figure 3. The variable capacitor C1 (42) together with C2 (40) and resistors R1 (34) and R2 (36) in Figure 4 are represented as crystal trim 206 in Figure 3. The chip MM5369, shown as numeral 32 in Figure 4, is represented as clock-pulse generator 208 in Figure 3.

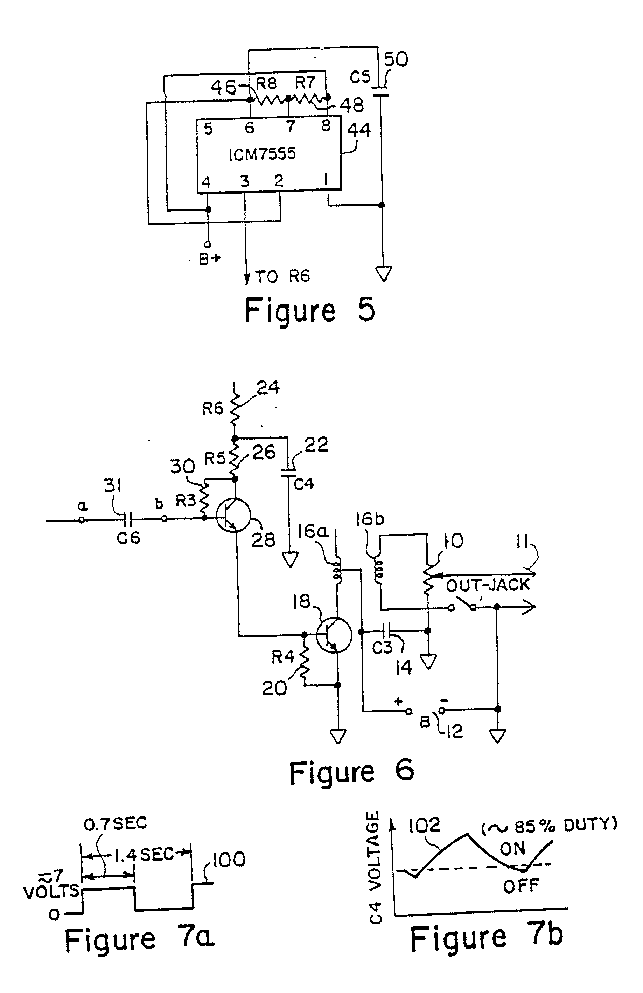

The circuit illustrated in Figure 5 is used to generate the voltage shown in Figure 7a (100) and has an output pin 3. This cycling pulse generator uses a conventional timing chip 44, typified by commercially available chip ICM7555. The repetition rate of the output from pin 4 is determined by resistors R8 (46), R7 (48), and capacitor C5 (50) . In the present invention, this voltage 100 has a typical amplitude of 7 volts, on and off for equal durations, and a pulse repetition rate of, typically, 1.4 seconds. Chip 44 in Figure 5 is represented as cycling pulse generator 202 in Figure 3 while resistors R8 (46), R7 (48), and capacitor C5 (50) are represented as chip circuitry 200 in Figure 3.

The circuit illustrated in Figure 6 is used to generate the output waveform 106 shown in Figure 10. The waveform network uses a pair of NPN transistors 28 and 18 connected as a modified Darlington pair. The clock pulse 104 of Figure 8 is coupled via capacitor C6 (31) to the base of transistor 28. Resistor R3 (30) serves in base bias position. Voltage 100 of Figure 7a is coupled to the collector of transistor 28 through an RC network comprising resistors R6 (24), R5 (26), and capacitor C4 (22). The time constant of this RC network is chosen to produce the sawtooth waveform 102 illustrated in Figure 7b. This waveform turns transistor 28 on and off with approximately an 85% to 90% duty cycle.

Transistor 18 stands in emitter-follower position to transistor 28. Resistor R4 (20) places the transistor 28 output across base-emitter of transistor 18.

The collector output of transistor 18 is coupled via transformer with primary winding 16a and secondary winding 16b. Capacitor C3 (14) provides bypassing. The gradually increasing, positive portion of the leading edge of the clock pulse after C6 (Figure 9) results in gradually increasing currents and voltages in the transformer windings, the voltages being proportional to the rate of change of current with time ($V = L\, di/dt$). At the sharp trailing edge of the clock pulse the current shut-down is very sudden, resulting in an induced voltage 105 greater than the first, of shorter duration, and opposite polarity. The typical output waveform 106 (Figure 10), before control by potentiometer 10 (Figure 6), before losses in transmission lines to electrodes, and before losses in coupling between electrode and patient, has a pulse width of 1 millisecond, a positive pulse amplitude of 20 volts, a negative spike amplitude of minus 70 volts, a pulse repetition rate of 100 Hertz, and is gated on and off with a duty cycle of about 85%. The waveform network illustrated in Figure 6 is represented by waveform network 210 in Figure 3.

The output of transformer secondary 16b is coupled through potentiometer 10 to a switched output jack 11 so that the electrode harness 300 (Figure 2) can be plugged in. The negative connection of battery 12 is switched by output jack 11 so that power is only supplied to the device when a plug is inserted into output jack 11.

Figures 3 to 10 throughout, and particularly Figure 6, distinguish between a chassis ground and a battery ground (negative) which are made common to place the circuit in operation. These grounds are common points internal to the circuitry and they are never connected to an outside grounding system. From the point of view of the patient, there is nothing to distinguish between the two leads from the circuitry to the electrodes except that they carry waveforms of relatively opposite polarity. When a waveform of one polarity is applied to one mastoid process, a waveform of opposite polarity is applied to the other.

Figure 11 shows in block form a typical assortment of monitoring functions to assist in standardized administration of patient care. Final specification of functions to be monitored - and implementing circuitry - await completion and evaluation of current clinical trials. They will be of standard type, but are illustrated since the present application concerns the complete clinical method.

While certain novel features of this invention have been shown and described and are pointed out in the annexed claims, it will be understood that various omissions, substitutions and changes in the forms and the details of the device illustrated and in its operation can be made by those skilled in the art without departing from the spirit and scope of the invention.

## Claims

1. A method of relieving a patient's suffering due to narcotic and alcohol withdrawal, using electrical pulses characterized by:
   a. applying a train of electrical pulses (106) to the patient's mastoid processes (306); and
   b. controlling the waveform and frequency of said pulses.

2. A method as defined in Claim 1, wherein said electrical pulses (106) have a typical pulse width of 1 millisecond and a typical amplitude, at the source, of 20 volts and lower, and a typical pulse repetition rate of 100 Hertz.

3. A method as defined in Claim 1, wherein said method comprises the step of applying said train of electrical pulses (106) to the patient's mastoid processes (306), wherein said electrical pulses (106) comprise of square waves (106a) and spikes (106b) and are applied to each of said mastoid processes (306) simultaneously, said square waves (106a) and spikes (106b), when applied to each of said mastoid processes (306) being of opposite polarity, said square wave electrical pulses (106a) having a pulse width of 1 millisecond and an amplitude, at the source, of 20 volts and lower, and a pulse repetition rate of 100 Hertz, said electrical spikes (106b) having an amplitude, at the source, of 70 volts with a polarity opposite to said square wave pulses (106a) wherein a pulse spike (106b) follows immediately upon the conclusion of each of said square wave pulses (106a), and controlling the waveform and frequency of said pulses.

4. A method as defined in Claim 2, wherein each of said electrical pulses (106) further comprises an electrical spike (106b) with a typical amplitude, at the source, of 70 volts with a polarity opposite to said pulse of Claim 2, wherein a spike (106b) follows immediately upon the conclusion of each of said pulses (106).

5. A drug-free neurostimulator characterized by having:
   a. electrode means (302, 304) for applying a train of electrical pulses (106) to the mastoid processes (306) of a patient;
   b. means (200, 202, 204, 206, 208, 210) for generating a train of electrical pulses (106) to be input to said electrode means (302, 304), wherein, by the application of said pulses (106) to said mastoid processes (306), a patient's suffering due to narcotic and alcohol withdrawal is relieved.

6. A neurostimulator as defined in Claim 5, wherein said electrode means comprises electrodes (302, 304) for applying said train of electrical pulses (106) to the mastoid processes (306) of a patient, said train of electrical pulses (106) comprising individual pulses (106a) with an amplitude of 20 volts and a pulse width of 1 millisecond, followed immediately by a voltage spike (106b) of opposite polarity with an amplitude of 70 volts, wherein said pulses (106) have a pulse repetition rate of 100 Hertz, with both voltage magnitudes measured at the source; an electrode harness (300) being in electrical communication with said electrodes (302, 304) and carrying said train of electrical pulses (106) to said electrodes (302, 304); and wherein said train of electrical pulses (106) is carried by said electrode harness (300) to said electrodes (302, 304) so that application of said electrical pulses (106) is provided to said mastoid processes (306).

7. A neurostimulator as defined in Claim 5, wherein said train of electrical pulses (106) comprises individual pulses (106a) with typical amplitude of 20 volts and typical pulse width of 1 millisecond, followed immediately by a voltage spike (106b) of opposite polarity with typical amplitude of 70 volts, wherein said pulses (106a) have a typical pulse repetition rate of 100 Hertz, with both voltage magnitudes measured at the source.

8. A neurostimulator as defined in Claim 6, wherein said means (200, 202, 204, 206, 208, 210) for generating a train of electrical pulses (106) carried by said electrode harness (300) to said electrodes (302, 304), comprises a crystal-controlled oscillator and divider (32) to generate positive going pulses (106), with a pulse width of 1 millisecond and a pulse repetition rate of 100 Hertz, a cycling-pulse generator (202) to generate a square wave (100) with a pulse width of 0.7 seconds, and a waveform network (210) whose inputs are said positive going pulses (104) and said square wave (100), and whose output is a pulse train (106) comprising pulses (106a) with a pulse width of 1 millisecond and an amplitude of 20 volts, followed immediately by a spike (106b) of opposite polarity with an amplitude of 70 volts; wherein said pulse train (106) is alternately turned on and off with a duty cycle of 85%.

9. A neurostimulator as defined in Claim 8, wherein said waveform network (210) comprises a transistor pair (28, 18) wherein positive clock pulses (104) are coupled to the base input of a first stage, said square wave (100) is coupled via a resistor-capacitor network to the collector of said first stage, and the output of a second stage is transformer-coupled to said electrode harness (300), such that both waveform network inputs present a high impedance to reduce waveform distortion, while the output of the final stage is presented to said electrode harness (300).

10. A neurostimulator as defined in Claim 9, further comprising a potentiometer (10) connected between the output of said waveform network (210) and said electrodes (302, 304), wherein said potentiometer (10) is used to vary the intensity of stimulation of said patient's mastoid processes (306).

Figure 1

Figure 2

305

302

308

**Figure 2a**

316

302

**Figure 2b**

```
  ┌─200          ┌─202
┌──────────┐   ┌──────────┐
│   CHIP   │   │ CYCLING  │
│ CIRCUITRY│──▶│  PULSE   │
│          │   │GENERATOR │
└──────────┘   └──────────┘
   ┌─208            │ ┌─210
┌──────────┐   ┌──────────┐
│  CLOCK-  │   │ WAVEFORM │  OUT
│  PULSE   │──▶│ NETWORK  │──▶
│GENERATOR │   │          │
└──────────┘   └──────────┘
     ▲
┌──────────┐
│ CRYSTAL  │
│   TRIM   │──206
│          │
└──────────┘
     ▲
┌──────────┐
│          │──204
│ CRYSTAL  │
│          │
└──────────┘
```

**Figure 3**

a    TO C6

MM5369    ~32

8        6        5

R1  ~36
34
38   R2

B+

C1        C2
42        40

**Figure 4**

8

EP 0 408 783 A1

Figure 5

Figure 6

Figure 7a

Figure 7b

9

Figure 8

Figure 9

Figure 10

Figure 11

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| E | US-A-4865048 (H.D. ECKERSON) <br> * column 4, line 39 - column 5, line 52; claims 1-7; figures * | 1-10 | A61N1/36 |
| X | WO-A-8700063 (ZION FOUNDATION) <br> * page 16, line 31 - page 18, line 11; figures * <br> * page 21, line 29 - page 22, line 21; claims 1, 7, 35 * | 1-3, 5 | |
| X | GB-A-2052994 (J.J. COHEN) <br> * page 4, line 94 - page 7, line 25; figures * | 1, 2, 5, 10 | |
| A | US-A-3946745 (W. HSIANG-LAI ET AL.) <br> * column 1, lines 9 - 55; figures * <br> * column 3, line 61 - column 6, line 43 * | 1-10 | |
| A | FR-A-2342082 (R.E.M.P.A.C. S.C.) <br> * page 2, lines 15 - 39; figures * <br> * page 4, lines 8 - 16 * | 1-3, 5, 6 | |
| A | FR-A-2502015 (G.P. BENOIT) <br> * page 3, lines 4 - 7; figures 2a, 2c * | 1-7 | TECHNICAL FIELDS SEARCHED (Int. Cl.5 ) <br><br> A61N |

The present search report has been drawn up for all claims

| Place of search | Date of completion of the search | Examiner |
|---|---|---|
| THE HAGUE | 20 MARCH 1990 | RIEB K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document